# EUROPEAN PATENT SPECIFICATION

(11) **EP 3 266 397 B1**
(45) Date of publication and mention of the grant of the patent: **13.12.2023**
(21) Application number: 15884038.9
(22) Date of filing: 25.12.2015
(51) Int. Cl.: A61B 18/12, A61B 18/00, A61B 17/32

(54) **POWER SOURCE DEVICE FOR HIGH-FREQUENCY TREATMENT TOOL, HIGH-FREQUENCY TREATMENT SYSTEM, AND CONTROL METHOD FOR POWER SOURCE DEVICE**
STROMQUELLENVORRICHTUNG FÜR EIN HOCHFREQUENZBEHANDLUNGSWERKZEUG, HOCHFREQUENZBEHANDLUNGSSYSTEM UND STEUERUNGSVERFAHREN FÜR DIE STROMQUELLENVORRICHTUNG
DISPOSITIF DE SOURCE D'ÉNERGIE POUR INSTRUMENT DE TRAITEMENT HAUTE FRÉQUENCE, SYSTÈME DE TRAITEMENT HAUTE FRÉQUENCE, ET PROCÉDÉ DE COMMANDE POUR UN DISPOSITIF DE SOURCE D'ÉNERGIE

(30) Priority: 02.03.2015 JP 2015040045
(43) Date of publication of application: 10.01.2018
(73) Proprietor: Olympus Corporation, Hachioji-shi, Tokyo 192-8507 (JP)
(72) Inventor: HONDA, Satoshi, Hachioji-shi, Tokyo 192-8507 (JP); HAYASHIDA, Tsuyoshi, Hachioji-shi, Tokyo 192-8507 (JP)
(74) Representative: Gunzelmann, Rainer
(86) International application number: PCT/JP2015/086400
(87) International publication number: WO 2016/139872

(56) References cited:
- EP-A1- 0 219 568
- EP-A1- 2 777 581
- WO-A2-93/03677
- JP-A- S5 335 287
- JP-A- H09 154 850
- JP-A- 2008 519 620
- JP-B1- 5 526 296
- JP-B2- 5 544 054
- JP-U- H0 430 509
- US-A- 4 860 745

## Description

### Technical Field

The present invention relates to a power supply device for a high-frequency treatment instrument and a high-frequency treatment system.

### Background Art

A treatment instrument used for high-frequency treatment, which applies a high-frequency current to living tissue which is a target of treatment, and heats the living tissue to perform treatment, is known. It is desired to accurately control the output of such a high-frequency treatment instrument.

International Patent Application Publication No. 2010/064530 A1, for example, discloses a technique to prevent the high-frequency treatment instrument from exhibiting unintended behaviors in a manner described below. In other words, the high-frequency treatment instrument obtains impedance of a circuit and a DC component of a waveform of a high-frequency voltage. If the impedance falls under a predetermined range, the output is controlled in such a manner that the DC component does not exceed a predetermined threshold. As a result, the high-frequency treatment instrument does not exhibit an intended behavior.

As a further example, U.S. Patent Application Publication No. 4 860 745 A discloses a high frequency electrosurgical apparatus for coagulation of a biological tissue wherein, as electric arcs are generated between a coagulation electrode and a target tissue of a patient, because of a nonlinear behavior of the electrical resistance generating the arc, and because of a stochastic frequency of the arc per unit of time, anharmonic frequencies are generated among harmonic frequencies. The generation of anharmonic frequencies during coagulation process may be used as a criterion for determining automatic shutoff of a current. More in detail, an arc monitor is provided with a filter which selectively passes a specific anharmonic frequency or a more or less wide frequency spectrum between two adjacent harmonic frequencies, or a plurality of more or less wide frequency spectra of the anharmonic frequencies between various adjacent harmonic frequencies of a base frequency. Then, an output signal from the filter is supplied to a voltage comparator which outputs a signal to a bistable circuit, so that the bistable circuit may provide a shutoff signal to a line, and thereby determine the current to be shut off.

Moreover, European Patent Application Publication No. 2 777 581 A1 discloses a technique for detecting arc patterns between an electrosurgical instrument and a living tissue during a surgical procedure, wherein a voltage sensor and a current sensor are configured to measure a voltage waveform and a current waveform during arching, respectively. More in detail, the current waveform shows distinct harmonic distortion, while the voltage waveform shows little distortion. A controller may be configured so as to detect arching while detecting the harmonic distortion or a particular shape or other characteristic of the harmonic distortion in the current waveform. Alternatively, the controller may be configured to detect arching while detecting the harmonic distortion or a particular shape or other characteristic of the harmonic distortion in the voltage waveform.

In addition, European Patent Application Publication No. EP 0 219 568 A1 discloses a high-frequency surgical device in which, when electrical arcs are generated between an active electrod and a treated living tissue, because of the nonlinear behavior of the electrical resistance generating the arcs, and because of a stochastic frequency of the arcs per unit of time, non-harmonic frequencies are generated among harmonic frequencies. Since the non-harmonic frequencies caused by the arcs are stochastic, their distribution is uninterrupted. The non-harmonic frequencies that are suitable for controlling intensity of the electric arcs are filtered by a filter and then supplied to a rectifier via a line. Subsequently, a rectified result is fed from the rectifier to a control amplifier.

Still further, International Patent Application Publication No. 93/03677 A2 discloses a surgical high-frequency generator for performing high-frequency surgical cutting, wherein two filters for evaluating spectral fractions of a generator signal may be provided in an indicator device. Specifically, the first filter is configured to determine a spectral performance with one or several odd harmonics of a basic frequency of the generator, while the second filter is configured to determine a spectral performance with one or several even harmonics of the basic frequency of the generator. A timer may be further provided for lowering power from the generator for a predetermined time such that, during the predetermined time, generation of arcs may be prevented and unnecessary supply of high power to the tissue may be avoided.

### Summary of Invention

In a high-frequency treatment instrument, an unintended excessive current sometimes flows when, for example, an electrode touches metal such as a forceps. Controlling the output of the high-frequency treatment instrument with as high accuracy level as possible is desired.

The purpose of the present invention is to provide a power supply device for a high-frequency treatment instrument and a high-frequency treatment system, which can suppress an excessive output.

According to an aspect of the present invention, a power supply device for a high-frequency treatment instrument which performs high-frequency treatment on living tissue includes the features of claim 1. A power supply device may include an output section which outputs power having a first frequency supplied to the high-frequency treatment instrument; a characteristic detector which detects a characteristic signal generated before the power outputted from the output section becomes excessive, the characteristic signal having a second frequency higher than the first frequency and being included in a signal related to the power; and a stop signal generator which outputs a stop signal to stop or reduce an output of the power from the output section when the characteristic signal is detected.

According to an aspect of the present invention, a high-frequency treatment system includes the above-mentioned power supply device; and the high-frequency treatment instrument connected to the power supply device.

According to a comparative example, a control method of a power supply device for a high-frequency treatment instrument which performs high-frequency treatment on living tissue may include outputting power having a first frequency supplied to the high-frequency treatment instrument; detecting a characteristic signal generated before the power becomes excessive, the characteristic signal having a second frequency higher than the first frequency and being included in a signal related to the power; and outputting a stop signal to stop or reduce an output of the power when the characteristic signal is detected.

According to the present invention, a power supply device for a high-frequency treatment instrument and a high-frequency treatment system which can suppress an excessive output can be provided.

### Brief Description of Drawings

FIG. 1 is a block diagram showing an outline of the configuration example of a high-frequency treatment system according to the first embodiment;
FIG. 2 is a block diagram showing an outline of the exterior of the configuration example of a high-frequency treatment system according to the first embodiment;
FIG. 3 is a block diagram showing an outline of the configuration example of an electric parameter detector, a characteristic detector, and a stop signal generator according to the first embodiment;
FIG. 4 is a flowchart illustrating an outline of the operation of a power supply device according to the first embodiment;
FIG. 5 is a diagram illustrating an outputted high-frequency current;
FIG. 6 is a diagram illustrating an outputted high-frequency current;
FIG. 7 is a diagram illustrating an outputted high-frequency current;
FIG. 8 is a diagram illustrating a first high-frequency current after passing a first low-pass filter;
FIG. 9 is a diagram illustrating the switching of an output when a characteristic current is detected;
FIG. 10 is a drawing to explain advantages of the first embodiment;
FIG. 11 is a block diagram showing an outline of the configuration example of a characteristic detector according to the first modification of the first embodiment;
FIG. 12 is a block diagram showing an outline of the configuration example of a characteristic detector according to the second modification of the first embodiment;
FIG. 13 is a diagram illustrating a high-frequency current and an output from a comparator according to the second modification of the first embodiment;
FIG. 14 is a block diagram showing an outline of the configuration example of a characteristic detector according to the third modification of the first embodiment;
FIG. 15 is a diagram illustrating a high-frequency current and an output from a comparator according to the third modification of the first embodiment;
FIG. 16 is a diagram illustrating switching of an output when a characteristic current is detected according to the fourth modification of the first embodiment;
FIG. 17 is a diagram illustrating switching of an output when a characteristic current is detected according to the fifth modification of the first embodiment;
FIG. 18 is a block diagram showing an outline of the configuration example of a high-frequency treatment system according to the sixth modification of the first embodiment;
FIG. 19 is a block diagram showing an outline of the configuration example of an electric parameter detector, a characteristic detector, and a stop signal generator according to the second embodiment;
FIG. 20 is a drawing to explain a high-frequency current after passing a band-pass filter according to the second embodiment;
FIG. 21 is a block diagram showing an outline of the configuration example of a high-frequency treatment system according to the third embodiment; and
FIG. 22 is a flowchart illustrating an outline of the operation of a power supply device according to the third embodiment.

The method steps described below only represent background that is useful for understanding the present invention, but do not form part of the same.

### Description of Embodiments

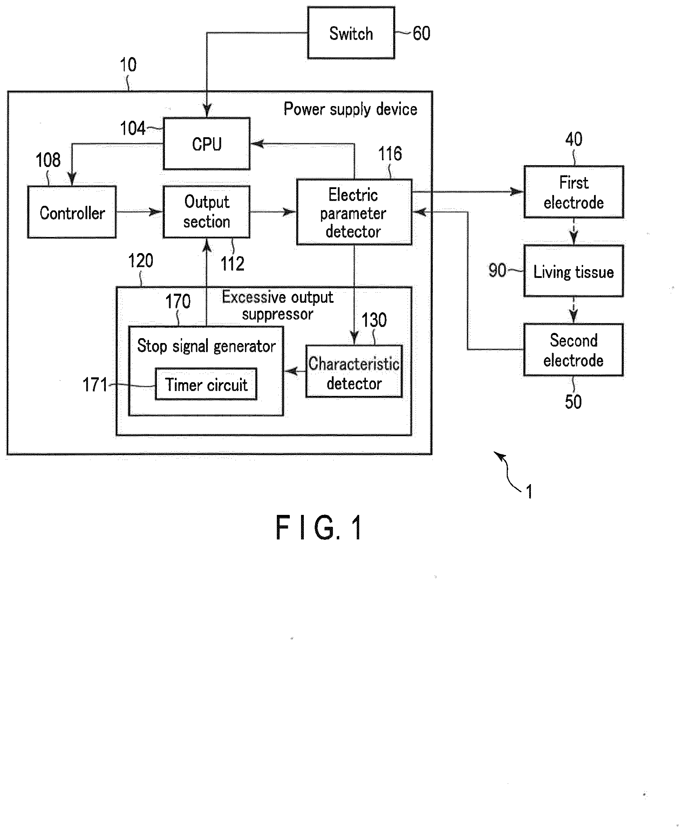

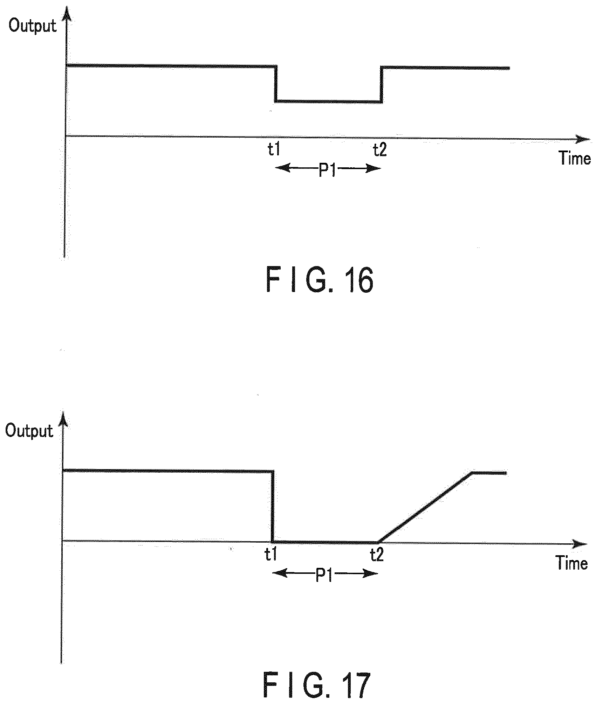

The first embodiment described with reference to FIGS. 1 to 10 is an example useful for understanding the invention. The modification described with reference to FIG. 16 is an example useful for understanding the invention. An implementation of the invention is described in the fifth modification of the first embodiment with reference to FIGS. 1 to 8, 10 and 17.

### [First Embodiment]

The first embodiment of the present invention will be described with reference to the drawings. FIG. 1 is a block diagram showing an outline of the configuration example of a high-frequency treatment system 1 according to the first embodiment. As shown in FIG. 1, the high-frequency treatment system 1 comprises a first electrode 40 and a second electrode 50 to apply a high-frequency current to living tissue 90 which is a treatment target. The high-frequency treatment system 1 comprises a power supply device 10 to supply power to the first electrode 40 and the second electrode 50, and a switch 60 operated by an operator to switch between on and off for the power supply from the power supply device 10 to the first electrode 40 and the second electrode 50.

The power supply device 10 comprises a central processing unit (CPU) 104, a controller 108, an output section 112, an electric parameter detector 116, and an excessive output suppressor 120.

The controller 108 controls the power supply to the first electrode 40 and the second electrode 50. The controller 108 includes a Central Processing Unit (CPU), an Application Specific Integrated Circuit (ASIC) or the like. The controller 108 outputs information regarding the on and off of the output and information regarding a current or a voltage to be output to the output section 112.

The output section 112 outputs power under the control of the controller 108. In other words, the output section 112 applies a voltage designated by the controller 108 to a circuit which includes the first electrode 40 and the second electrode 50, and feeds a current designated by the controller 108 to the circuit. The output power is supplied to the first electrode 40 and the second electrode 50 via the electric parameter detector 116.

The electric parameter detector 116 detects the output from the output section 112. The electric parameter detector 116 retrieves as output information a voltage applied to the first electrode 40 and the second electrode 50, and a signal related to a current fed to the first electrode 40 and the second electrode 50.

The CPU 104 controls the operation of each section of the power supply device 10. For example, the CPU 104 detects an input of an instruction indicating that an output should be performed to the switch 60, and outputs information indicating that the power supply device 10 should perform the output and information, such as output intensity, etc., to the controller 108. The power supply device 10 performs feedback control regarding the output. For this reason, the CPU 104 obtains information regarding a present output from the electric parameter detector 116, and determines an output to be performed thereafter based on the information, etc.

The excessive output suppressor 120 prevents an output from the power supply device 10 from becoming excessive. The excessive output suppressor 120 monitors an output to detect signs of an excessive output, and has the output section 112 stop the power supply when such a sign is detected. The excessive output suppressor 120 comprises a characteristic detector 130 and a stop signal generator 170.

The characteristic detector 130 obtains output information from the electric parameter detector 116, and detects from the information characteristic changes that are developed in a current or a voltage before an excessive output is performed. When a characteristic change in a current or a voltage is detected, the characteristic detector 130 informs a stop signal generator 170 of the detection.

The stop signal generator 170 has a timer circuit 171. When the stop signal generator 170 obtains information indicating that a characteristic change is detected in the current or the voltage from the characteristic detector 130, the stop signal generator 170 outputs an output stop signal to the output section 112 indicating that the output should be stopped. Herein, the stop signal generator 170 outputs the output stop signal to the output section 112 only for a period measured by the timer circuit 171.

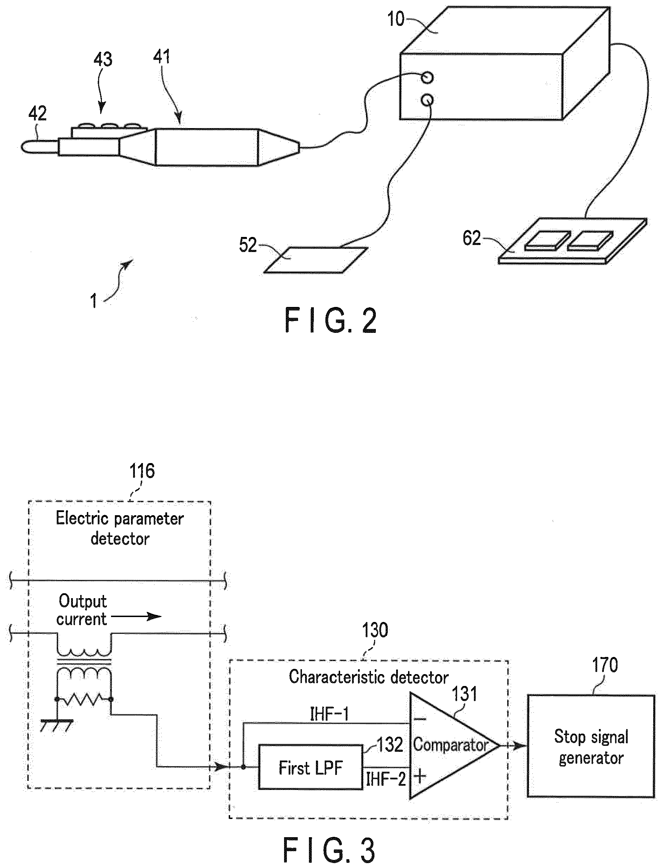

FIG. 2 shows one example of the exterior of the high-frequency treatment system 1 including a monopolar high-frequency treatment instrument as an example of the high-frequency treatment system 1 shown in FIG. 1. As shown in FIG. 2, the high-frequency treatment system 1 comprises a high-frequency treatment instrument 41 to coagulate or cut the living tissue 90. The high-frequency treatment instrument 41 has an electrode 42 that functions as the first electrode 40. The high-frequency treatment instrument 41 has an output switch 43 that functions as the switch 60. The output switch 43 includes a switch between on and off for the output in the cut mode, and a switch between on and off for the output in the coagulation mode. The high-frequency treatment system 1 also comprises a return pad 52 that functions as the second electrode 50. The high-frequency treatment system 1 comprises a foot switch 62 having a function similar to the output switch 43. The high-frequency treatment instrument 41, the return pad 52, and the foot switch are connected to the power supply device 10.

During the treatment, the return pad 52 is attached to the body of a subject person. The operator holds the high-frequency treatment instrument 41, and turns the power supply on using the output switch 43 or the foot switch 62, having the electrode 42 being touched with a site targeted for cutting or coagulation. As a result, cutting or coagulation of the living tissue is performed at a treatment target site.

Herein, the monopolar high-frequency treatment instrument is described as an example; however, the technique according to the present embodiment may be applied to a bipolar high-frequency treatment instrument in a similar manner. In a case of a bipolar high-frequency treatment instrument, for example, one of a pair of holding members of a forceps-shaped treatment instrument is configured to function as the first electrode 40, and the other is configured to function as the second electrode 50.

Next, an example of a specific configuration of the electric parameter detector 116 and the characteristic detector 130, which have been described with reference to FIG. 1, will be described with reference to FIG. 3.

The electric parameter detector 116, as shown in FIG. 3, retrieves a part of an AC current that is output from the output section 112 as a current used for detection, using a circuit into which a transformer and resistor are incorporated. The current used for detection is input to the characteristic detector 130.

A first low-pass filter (LPF) 132 and a comparator 131 are provided in the characteristic detector 130. The current used for detection, which is input to the characteristic detector 130, is bifurcated. One of the bifurcated current used for detection is referred to as a first high-frequency current IHF-1, and the other as a second high-frequency current IHF-2. The first high-frequency current IHF-1 is input to an inverting input terminal of the comparator 131. The second high-frequency current IHF-2 is input to the first LPF 132.

The first LPF 132 is a common low-pass filter comprised of electronic devices, such as a low-pass filter comprised of a resistor and a capacitor, a low-pass filter configured with an operational amplifier, and the like. High-frequency components higher than a predetermined frequency in the second high-frequency current IHF-2 are cut off by the first LPF 132. The second high-frequency current IHF-2 passing the first LPF 132 is input to non-inverting input terminal of the comparator 131.

A comparison signal is output from the output terminal of the comparator 131 in accordance with a result of comparison between the first high-frequency current IHF-1 and the second high-frequency current IHF-2. The comparison signal is input to the stop signal generator 170.

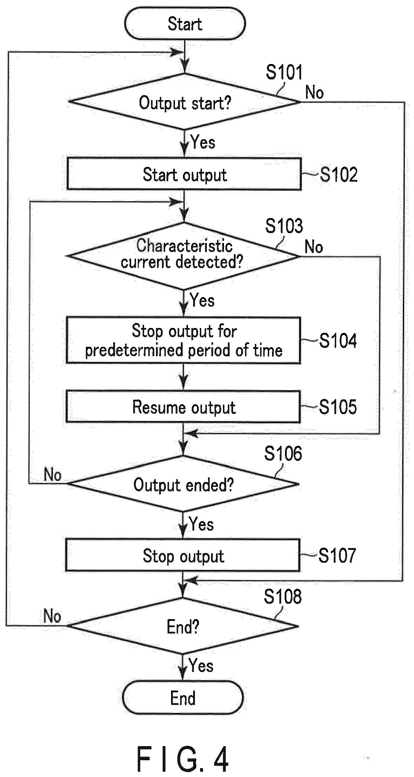

Next, the operation of the power supply device 10 according to the present embodiment will be described with reference to the flowchart shown in FIG. 4. The process shown in FIG. 4 is started when the power supply device 10 is turned on, for example.

In step S101, the power supply device 10 determines whether the output should be started or not. In other words, the CPU 104, for example, determines whether the switch 60 is turned on or not. If the output is not started, the process proceeds to step S108, and the process in step S101 is repeated to wait, unless the power source is turned off as will be described later. When the output is started, the process proceeds to step S102.

In step S102, the power supply device 10 starts the output. In other words, the CPU 104 outputs to the controller 108 information indicating that the output should be performed and information regarding an output current. The controller 108 controls the operation of the output section 112 based on the information obtained from the CPU 104. The output section 112 outputs a designated high-frequency current under the control of the controller 108. The outputted high-frequency current flows in the first electrode 40 and the second electrode 50 via the electric parameter detector 116, and in the living tissue 90 placed between the first electrode 40 and the second electrode 50. The living tissue 90 generates heat at a site where the living tissue 90 is in contact with, for example, the first electrode 40. The living tissue is, for example, cauterized by this heat.

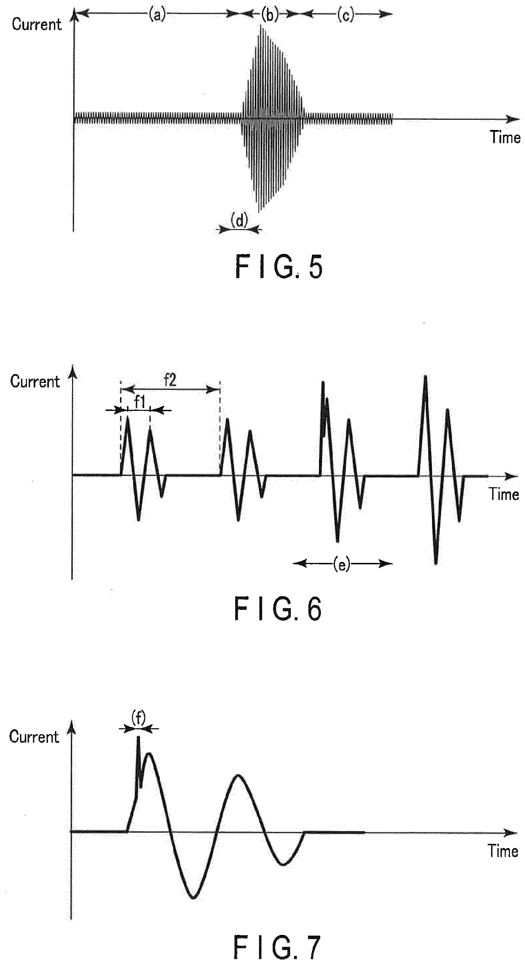

The current flowing at this time is described with reference to the schematic drawings of FIGS. 5 to 7. FIG. 5 is a drawing schematically showing an amplitude of a current with respect to time during a treatment. As in the period (a) in FIG. 5, a current value is retained within a certain range when the first electrode 40 and the second electrode 50 are in contact with the living tissue 90. However, when, for example, the first electrode 40, etc. touches a metallic object, etc. such as the forceps holding the living tissue 90, a current value may become extremely high as in the period (b) in FIG. 5. In other words, an excessive current flows. Such an excessive current eventually drops when the contact with a metallic object, etc. is eliminated, and returns to a constant current as in the period (c) in FIG. 5, like at the case before the touching the metallic object. A flow of an excessive current as in the period (b) in FIG. 5 is undesirable because it means that an actual current value is different from a control target value. The output of such an excessive current is suppressed in the present embodiment.

FIG. 6 shows a relationship between the time and current in the period (d) in FIG. 5 in an expanded time base. As shown in FIG. 6, in the present embodiment, an AC current having a fundamental frequency corresponding to a cycle period indicated as f1 is consecutively output, and intermittently output for each repeated frequency in a cycle period indicated as f2. A fundamental frequency is a frequency of 400 to 500 kHz, for example. The current value gradually increases in the period shown in FIG. 6.

FIG. 7 shows a relationship between the time and current in the period indicated as (e) in FIG. 6 in an expanded time base. As shown in FIG. 7, in the period (f), a characteristic waveform having a frequency higher than the fundamental frequency is observed. Hereinafter, a current having such a characteristic waveform will be referred to as a characteristic current. The frequency of a characteristic current is, for example, 6 MHz or so. In the present embodiment, a characteristic current is detected to suppress the output of an excessive current.

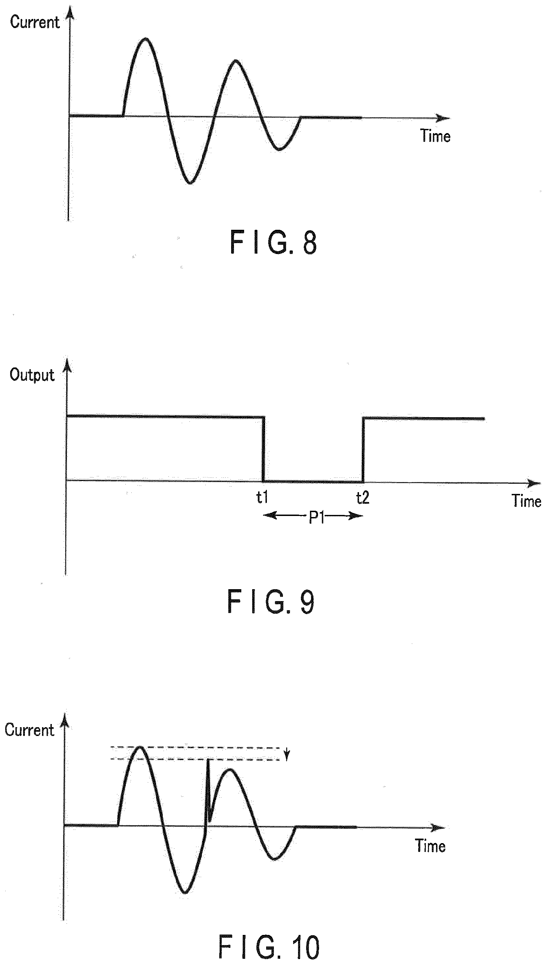

The explanation continues, returning to FIG. 4. In step S103, the power supply device 10 determines whether or not a characteristic current is detected. In other words, in the characteristic detector 130, the first high-frequency current IHF-1 related to an output current is input to the inverting input terminal of the comparator 131. The second high-frequency current IHF-2, which is the same as the first high-frequency current IHF-1 and in which the high-frequency component is cut by the first LPF 132, is input to the non-inverting input terminal of the comparator 131. Herein, the cutoff frequency of the first LPF 132 is set lower than the characteristic current and higher than the fundamental current. Accordingly, if the output current does not include a characteristic current, there is no difference between a signal which is input to the inverting input terminal of the comparator 131 and a signal which is input to the non-inverting input terminal of the comparator 131. On the other hand, if the output current includes a characteristic current as shown in FIG. 7, a signal which is input to the inverting input terminal of the comparator 131 looks like the signal shown in FIG. 7, but a signal which is input to the non-inverting input terminal of the comparator 131 looks like the signal shown in FIG. 8. In other words, there is a difference between a signal which is input to the inverting input terminal and a signal which is input to the non-inverting input terminal. Accordingly, a comparison signal which is output from the output terminal of the comparator 131 is different depending on the presence/absence of a characteristic current. The stop signal generator 170 determines the presence/absence of a characteristic current based on the change in the comparison signal. If no characteristic current is detected, the process proceeds to step S106. On the other hand, if a characteristic current is detected, the process proceeds to step S104.

Thus, the first high-frequency current IHF-1 corresponding to a first signal related to the output current is compared with the second high-frequency current IHF-2 corresponding to a second signal in which the high-frequency component is cut by the first LPF 132, and when a signal value of the first signal is greater than a signal value of the second signal, it is determined that a characteristic signal is detected.

In step S104, the power supply device 10 stops the output only for a predetermined period of time. In other words, the stop signal generator 170 generates a stop signal, and outputs the stop signal to the output section 112. The period during which the stop signal is output is a predetermined period of time counted by the timer circuit 171. The predetermined period of time is a short period of time, such as 50 milliseconds to 100 milliseconds, for example, but is not limited thereto. The output section 112 which has received the stop signal stops the output only for the period during which the stop signal is received.

In step S105, the power supply device 10 resumes the output. In other words, the stop signal generator 170 stops outputting the stop signal after outputting the stop signal for the above-mentioned predetermined period. As a result, after stopping the output for the predetermined period, the output section 112 resumes the output. Accordingly, an outline of the output at the power supply device 10 looks like the drawing shown in FIG. 9. In other words, the output is stopped at time t1 shown in FIG. 9, and the output resumes at time t2 after the predetermined period P1 elapses.

In step S106, the power supply device 10 determines whether or not to end the output. In other words, the CPU 104 determines whether the switch 60 is turned off or not. If the power supply device 10 determines to not end the output, the process returns to step S103. In other words, the power supply device 10 repeats the above-described process from step S103 to step S106. When, on the other hand, the power supply device 10 determines to end the output, the process proceeds to step S107.

In step S107, the power supply device 10 stops the output. In other words, the CPU 104 outputs information indicating that the output should be stopped to the controller 108. The controller 108 controls the operation of the output section 112 based on the information obtained from the CPU 104 to stop the output.

In step S108, the power supply device 10 determines whether or not to end the process at the power supply device 10. If the power of the power supply device 10 is turned off, for example, the process at the power supply device 10 is ended. If the process is not ended, the process returns to step S101. In other words, the power supply device 10 repeats the above-described process from step S101 to step S108. When the process is ended, on the other hand, the repeated process is ended.

According to the present embodiment, the power supply device 10 detects a sign indicating a flow of an excessive current, and when the sign is detected, the output is stopped for a short period of time before an excessive current flows. As a result, a flow of an excessive current can be prevented. In other words, an excessive current like the one shown in the period (b) in FIG. 5 is not generated, and a stable current is retained as in the period (a) in FIG. 5. The power supply device 10 according to the present embodiment can thus control a current with high accuracy. Since the output resumes after a short period of output stoppage, it is possible to effectively suppress generation of an excessive current, with the output stoppage imposing a negligible influence on the treatment.

The characteristic detector 130 according to the present embodiment is comprised only of hardware, such as an operational amplifier, resistor, a capacitor, etc. For this reason, if a characteristic current is generated, a comparison signal output from the output terminal of the comparator 131 of the characteristic detector 130 changes in a few nanoseconds. This signal change is tremendously fast in comparison to the calculation using a CPU, for example, which takes a few milliseconds.

A method of detecting a characteristic current based on a difference between signals before and after passing a low-pass filter, like in the present embodiment, also increases accuracy in the characteristic current detection. In other words, when a characteristic current like the one in FIG. 7 is detected, it is possible to detect a characteristic current using a peak detection circuit. However, as shown in FIG. 10, for example, if a maximum value of a characteristic current is lower than a maximum value of a current waveform, the characteristic current cannot be detected depending on a peak detection circuit. On the other hand, the characteristic detector 130 according to the present embodiment can detect a characteristic current like the one shown in FIG. 10.

Herein, the output control at the power supply device 10 is performed by control of a current value. However, this is merely an example, and either control of a voltage value or control of a power value may be performed. An example has been described in which a disruption of control, caused by contact between an electrode and a forceps, is detected by monitoring a current value. However, the present invention is not limited to this example. A sign produced before the output becomes excessive can be detected as a characteristic signal by monitoring a voltage value and a power value.

### [First Modification of First Embodiment]

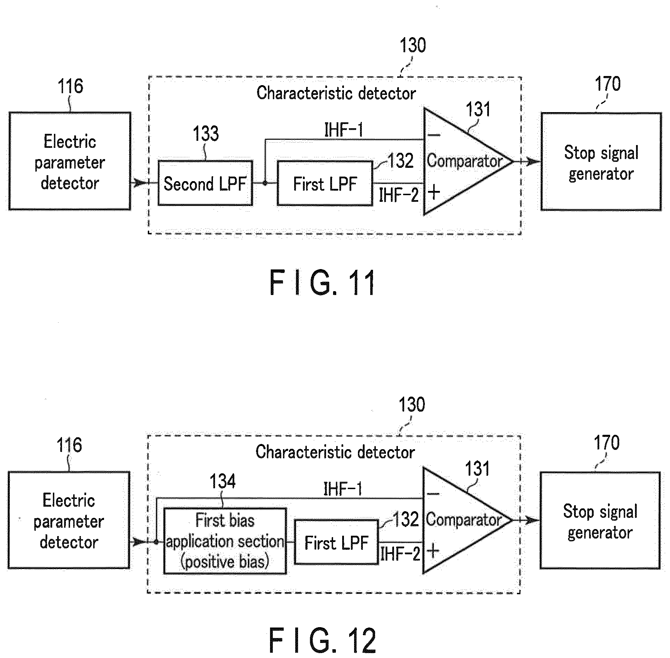

Next, a first modification of the first embodiment will be described. Herein, differences from the first embodiment will be described, and elements specified by the same reference numbers carry out the same operations, and a duplicate description of such elements will be omitted. In the present modification, the configuration of the characteristic detector 130 is different from that of the first embodiment. FIG. 11 illustrates the outline of a configuration example of the characteristic detector 130 according to the present modification.

As shown in FIG. 11, in the characteristic detector 130 of the present modification, a second low-pass filter (LPF) 133 is provided before a bifurcation of a current used for detection that is input from the electric parameter detector 116 so that the current passes the low-pass filter before being bifurcated into a first high-frequency current IHF-1 and a second high-frequency current IHF-2. The second LPF 133 is provided for the purpose of reducing the noise to prevent erroneous detection. Herein, the cutoff frequency of the second LPF 133 is higher than the frequency of the characteristic current. The configuration other than the above is the same as that of the first embodiment.

According to the present modification, since the noise having high-frequency components is reduced by the second LPF 133, the accuracy of detection is improved compared to the first embodiment. The present modification achieves the same advantageous effect as the first embodiment besides the above.

### [Second Modification of First Embodiment]

Next, a second modification of the first embodiment will be described. Herein, differences from the first embodiment will be described, and elements specified by the same reference numbers carry out the same operations, and a duplicate description of such elements will be omitted. In the present modification, the configuration of the characteristic detector 130 is different from that of the first embodiment. FIG. 12 illustrates the outline of an configuration example of the characteristic detector 130 according to the present modification.

As shown in FIG. 12, in the characteristic detector 130 of the present modification, a current used for detection that is input from the electric parameter detector 116 is bifurcated into a first high-frequency current IHF-1 and a second high-frequency current IHF-2. A first bias application section 134 is provided between the bifurcation and the first LPF 132 to apply a positive bias to a current value. Accordingly, the second high-frequency current IHF-2 passes the first LPF 132 after being positively biased. The configuration other than the above is the same as that of the first embodiment.

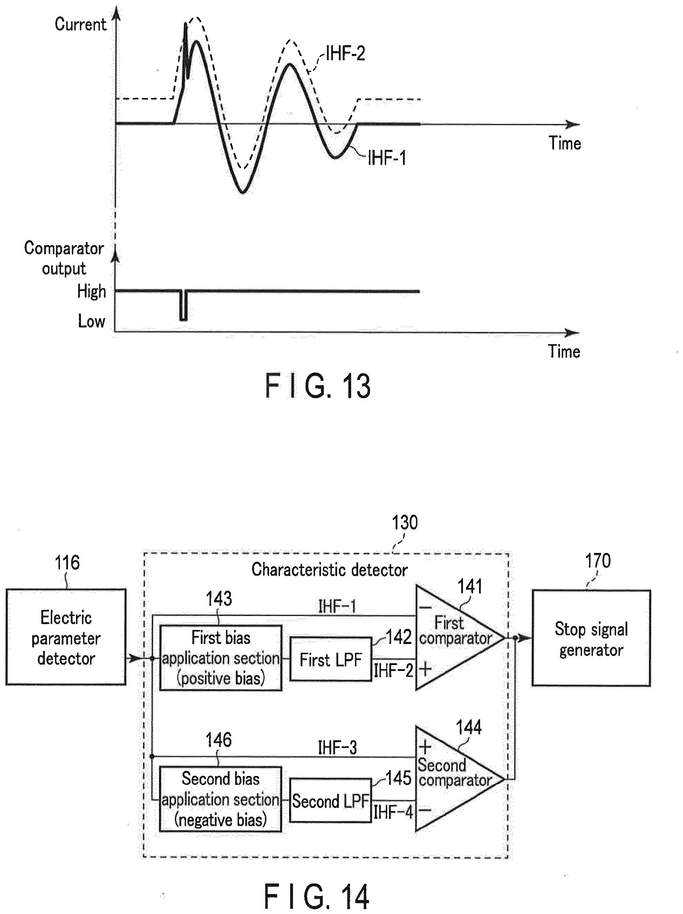

The current value and the output from the comparator 131 according to the present modification will be described with reference to the schematic drawing shown in FIG. 13. The top panel of FIG. 13 indicates the change in a current that is input to the comparator 131 with respect to time. In this drawing, the solid line schematically shows the change in the first high-frequency current IHF-1, and the broken line schematically shows the change in the second high-frequency current IHF-2. As shown in the drawing, the second high-frequency current IHF-2 indicated by the broken line is positively biased by the first bias application section 134. In the second high-frequency current IHF-2, the characteristic current, which is a high-frequency component, is cut off by the first LPF 132.

The first high-frequency current IHF-1 indicated by the solid line is input to the inverting input terminal of the comparator 131. On the other hand, the second high-frequency current IHF-2, which is indicated by the broken line and to which a positive bias is applied to cut a characteristic current, is input to the non-inverting input terminal of the comparator 131. Accordingly, if the value of the second high-frequency current IHF-2 is greater than the value of the first high-frequency current IHF-1, the comparison signal which is the output from the comparator 131 is at a high level. On the other hand, if the value of the first high-frequency current IHF-1 is greater than the value of the second high-frequency current IHF-2, the comparison signal which is the output from the comparator 131 is at a low level. Herein, when the amplitude of the characteristic current is greater than a bias value which is applied by the first bias application section 134, the output from the comparator 131 is at a low level at the time when the characteristic current is being generated, as shown in the bottom panel of FIG. 13.

In the present modification, the stop signal generator 170 outputs a stop signal when the comparison signal which is the output from the comparator 131 is at a low level.

Thus, the second high-frequency current IHF-2 corresponding to a third signal in which the characteristic current is cut off as a result of the application of a positive bias is compared with the first high-frequency current IHF-1 corresponding to a first signal, and when a signal value of the first signal is greater than a signal value of the third signal, it can be determined that a characteristic signal is detected.

According to the present modification, the characteristic detector 130 can perform the characteristic current detection more accurately than in the first embodiment. The present modification achieves the same advantageous effect as the first embodiment besides the above.

In the present modification, a positive bias is applied to the second high-frequency current IHF-2; however, it is the same if a negative bias is applied to the first high-frequency current IHF-1, instead. Similarly to the first modification, a low-pass filter for noise reduction may be provided in the present modification. The order of the first LPF 132 and the first bias application section 134 may be inverted.

Thus, the first high-frequency current IHF-1 corresponding to a fifth signal to which a negative bias is applied is compared with the second high-frequency current IHF-2 corresponding to a second signal to which a positive bias is not applied, and when a signal value of the fifth signal is greater than a signal value of the second signal, it can be determined that a characteristic signal is detected.

### [Third Modification of First Embodiment]

Next, a third modification of the first embodiment will be described. Herein, differences from the second modification of the first embodiment are described, and elements specified by the same reference numbers carry out the same operations, and a duplicate description of such elements will be omitted. In the present modification, the configuration of the characteristic detector 130 is different from that of the second modification of the first embodiment. In the second modification of the first embodiment, the characteristic detector 130 applies a positive bias to a second high-frequency current IHF-2 to detect a positive characteristic current. On the other hand, in the present modification, the characteristic detector 130 is configured to apply a negative bias as well to a second high-frequency current IHF-2 to detect a negative characteristic current. FIG. 14 illustrates the outline of an configuration example of the characteristic detector 130 according to the present modification.

As shown in FIG. 14, in the characteristic detector 130 of the present modification, a current used for detection that is input from the electric parameter detector 116 is quadfurcated into a first high-frequency current IHF-1, a second high-frequency current IHF-2, a third high-frequency current IHF-3, and a fourth high-frequency current IHF-4.

The characteristic detector 130 comprises a first comparator 141 and a second comparator 144. The first high-frequency current IHF-1 is input to the inverting input terminal of the first comparator 141. The characteristic detector 130 comprises a first bias application section 143 for applying a positive bias to the second high-frequency current IHF-2, and a first LPF 142 to cut high-frequency components of a characteristic current, etc. in the biased second high-frequency current IHF-2. The second high-frequency current IHF-2 is positively biased by the first bias application section 143, and is input to the non-inverting input terminal of the first comparator 141 after passing the first LPF 142.

The third high-frequency current IHF-3 is input to the non-inverting input terminal of the second comparator 144. The characteristic detector 130 comprises a second bias application section 146 for applying a negative bias to the fourth high-frequency current IHF-4, and a second LPF 145 to cut high-frequency components of a characteristic current, etc. in the biased fourth high-frequency current IHF-4. The fourth high-frequency current IHF-4 is negatively biased by the second bias application section 146, and is input to the inverting input terminal of the second comparator 144 after passing the second LPF 145.

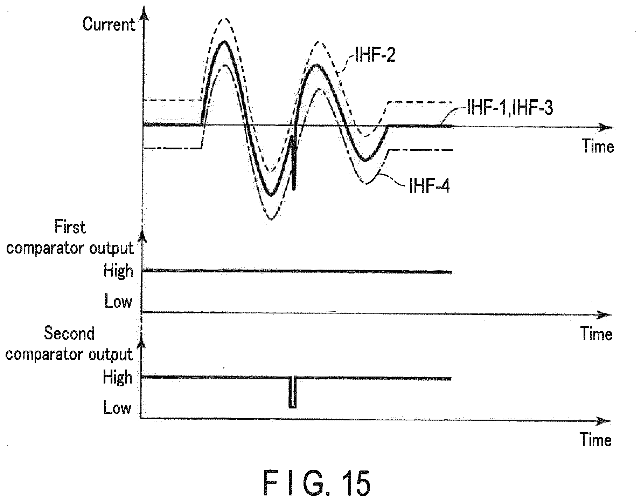

The current value and the output of the first comparator 141 and the second comparator 144 according to the present modification will be explained with reference to the schematic drawing of FIG. 15. The top panel of FIG. 15 indicates the change in a current that is input to the first comparator 141 and the second comparator 144 with respect to time. In this drawing, the solid line schematically shows the change in the first high-frequency current IHF-1 and the third high-frequency current IHF-3, the dashed line schematically shows the change in the second high-frequency current IHF-2, and the dashed-dotted line schematically shows the change in the fourth high-frequency current IHF-4. A negative characteristic current is generated in the first high-frequency current IHF-1 and the third high-frequency current IHF-3.

As shown in FIG. 15, the second high-frequency current IHF-2 indicated by the dashed line is positively biased by the first bias application section 143. In the second high-frequency current IHF-2, the characteristic current, which is a high-frequency component, is cut off by the first LPF 142. The fourth high-frequency current IHF-4 indicated by the dashed-dotted line is negatively biased by the second bias application section 146. In the fourth high-frequency current IHF-4, the characteristic current, which is a high-frequency component, is cut off by the second LPF 145.

The middle panel of FIG. 15 schematically shows the output of the first comparator 141, and the bottom panel of FIG. 15 schematically shows the output of the second comparator 144. Since the value of the second high-frequency current IHF-2 is always greater than the first high-frequency current IHF-1, the output from the first comparator 141 is always at a high level. On the other hand, since the value of the third high-frequency current IHF-3 is usually greater than the fourth high-frequency current IHF-4, the output from the second comparator 144 is always at a high level. Herein, when a negative characteristic current is generated, the fourth high-frequency current IHF-4 becomes greater than the third high-frequency current IHF-3; accordingly, when this occurs, the output of the second comparator 144 becomes at a low level.

The example shown in FIG. 15 is a case where the characteristic current is negative. If the characteristic current is positive, similar to the case of the second modification, the output of the first comparator 141 switches from the high level to the low level while the characteristic current is being generated.

Thus, the fourth high-frequency current IHF-4 corresponding to a fourth signal to which a negative bias is applied by the second bias application section 146 and passes the second LPF 145 is compared with the third high-frequency current IHF-3 corresponding to a first signal, and when a signal value of the first signal is lower than a signal value of the fourth signal, it can be determined that a characteristic signal is detected.

According to the present modification, the characteristic detector 130 can perform detection of a characteristic current more accurately than in the first embodiment, and the characteristic detector 130 can detect both a positive characteristic current and a negative characteristic current.

In the present modification, a positive bias is applied to the second high-frequency current IHF-2 and a negative bias is applied to the fourth high-frequency current IHF-4; however, it is the same if a negative bias is applied to the first high-frequency current IHF-1, and a positive bias is applied to the third high-frequency current IHF-3 instead. Similar to the first modification, a low-pass filter for noise reduction may be provided in the present modification.

Thus, the positively-biased third high-frequency current IHF-3 corresponding to a sixth signal is compared with the fourth high-frequency current IHF-4 corresponding to a second signal to which a negative bias is not applied, and when a signal value of the sixth signal becomes lower than a signal value of the second signal, it can be determined that a characteristic signal is detected.

### [Fourth Modification of First Embodiment]

Next, a fourth modification of the first embodiment will be described. Herein, differences from the first embodiment will be described, and elements specified by the same reference numbers carry out the same operations, and a duplicate description of such elements will be omitted. In the present modification, the operation related to stopping of the output at step S104 is different from that of the first embodiment. In other words, in the first embodiment, the output is stopped during a predetermined period P1 as shown in FIG. 9 when a characteristic current is detected. In contrast, in the present modification, as shown in FIG. 16, the output is reduced to half (the current value is halved) during the predetermined period P1 when a characteristic current is detected.

According to the present modification, a certain level of the output is retained when a characteristic current is measured, and the function as a high-frequency treatment instrument is also maintained at a certain level while suppressing an excessive current. The output in the predetermined period P1 may be any value and is not limited to a half value of the normal time. It should be noted, however, that the current value needs to be at a low level so that an excessive current can be suppressed.

The present modification is of course applicable not only to the first embodiment, but also to the first to third modifications of the first embodiment. Instead of lowering the current value, the present modification may be configured to switch between on and off during the predetermined period P1 to make the output intermittent.

### [Fifth Modification of First Embodiment]

Next, a fifth modification of the first embodiment will be described. Herein, differences from the first embodiment will be described, and elements specified by the same reference numbers carry out the same operations, and a duplicate description of such elements will be omitted. In the present modification, the operation related to resuming of the output at step S105 is different from that of the first embodiment. In other words, in the first embodiment, as shown in FIG. 9, the output current is reverted to the initial output immediately after the output is stopped for the predetermined period P1. On the other hand, in the present modification, as shown in FIG. 17, the output is gradually increased after the output is stopped for the predetermined period P1 and reverts to the initial output over time.

If the output is reverted to the initial level immediately after the output is stopped as in the first embodiment, the output at an excessive current may be caused at the time when the output resumes. On the other hand, according to the present modification, the output level is gradually increased, and if there is a possibility that the output at an excessive current occurs, a characteristic current is detected before the output at an excessive current occurs, and the output is prevented. As a result, the output at an excessive current is reliably prevented.

The present modification is of course applicable not only to the first embodiment, but also to the first to fourth modifications.

### [Sixth Modification of First Embodiment]

Next, a sixth modification of the first embodiment will be described. Herein, differences from the first embodiment will be described, and elements specified by the same reference numbers carry out the same operations, and a duplicate description of such elements will be omitted. The high-frequency treatment system 1 according to the present modification is configured to perform treatment using ultrasonic energy, in addition to treatment using high-frequency electric power.

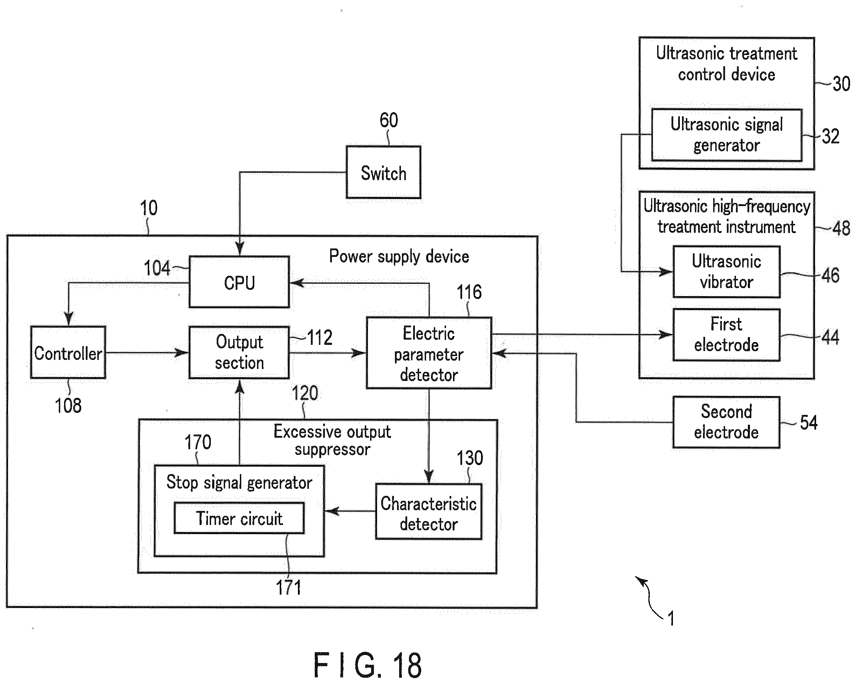

FIG. 18 illustrates the outline of the configuration example of the high-frequency treatment system 1 according to the present modification. As shown in FIG. 18, the configuration of the power supply device 10 and the switch 60 according to the present modification is the same as that of the power supply device 10 and the switch 60 according to the first embodiment. An ultrasonic high-frequency treatment instrument 48 is provided in the high-frequency treatment system 1 according to the present modification. The ultrasonic high-frequency treatment instrument 48 has a first electrode 44 corresponding to the first electrode 40 according to the first embodiment. Furthermore, the ultrasonic high-frequency treatment instrument 48 comprises an ultrasonic vibrator 46 which is a source of vibration for ultrasonically vibrating the first electrode 44. The ultrasonic treatment control device 30 for controlling the operation of the ultrasonic vibrator is provided in the high-frequency treatment system 1. The ultrasonic treatment control device 30 comprises an ultrasonic signal generator 32. The ultrasonic signal generator 32 generates a signal for driving the ultrasonic vibrator 46. The high-frequency treatment system 1 according to the present modification also comprises a second electrode 54 corresponding to the second electrode 50 according to the first embodiment. The second electrode 54 functions as a return pad.

The ultrasonic high-frequency treatment instrument 48 according to the present modification is brought in contact with living tissue, which is a treatment target to perform treatment by heat caused by supplying a high-frequency power and heat caused by ultrasonic vibration.

The operation as the high-frequency treatment device in the present modification is the same as in the first embodiment, and the same advantageous effect as in the first embodiment can be achieved. Furthermore, in the present modification, under the control of the ultrasonic treatment control device 30, the ultrasonic vibrator 46 vibrates ultrasonically and the vibration is transmitted to the first electrode 44. As a result, living tissue is treated also by the vibration of the first electrode 44. The operation of the power supply device 10 may of course be in conjunction with the operation of the ultrasonic treatment control device 30. For this reason, the power supply device 10 and the ultrasonic treatment control device 30 may be configured to exchange information.

The present modification is of course applicable not only to the first embodiment, but also to the first to fifth modifications.

The ultrasonic high-frequency treatment instrument 48 has, for example, a forceps-type shape, and a pair of holding members is the first electrode 44 and the second electrode 54, and may be configured to function as a pair of bipolar electrodes. In this case, one of the holding members in the pair vibrates at an ultrasonic frequency. In this case, the ultrasonic high-frequency treatment instrument 48 holds living tissue which is a treatment target, and performs treatment such as coagulation and cutting by heat caused by supplying high-frequency power, heat caused by ultrasonic vibration, and by mechanical power.

### [Second Embodiment]

A description will now be given of the second embodiment. Herein, differences from the first embodiment will be described, and elements specified by the same reference numbers carry out the same operations, and a duplicate description of such elements will be omitted. The configuration of the high-frequency treatment system 1 according to the second embodiment is different from the high-frequency treatment system 1 according to the first embodiment with respect to the configuration of the characteristic detector 130. In other words, the high-frequency treatment system 1 according to the first embodiment has the characteristic detector 130 bifurcate the inputted current used for detection, and one of the bifurcated currents used for detection is passed through the low-pass filter, and a comparator is provided for comparing the bifurcated current with the other bifurcated current used for detection that is not passed by the low-pass filter to detect a characteristic current. On the other hand, the characteristic detector 130 according to the present embodiment comprises a band-pass filter.

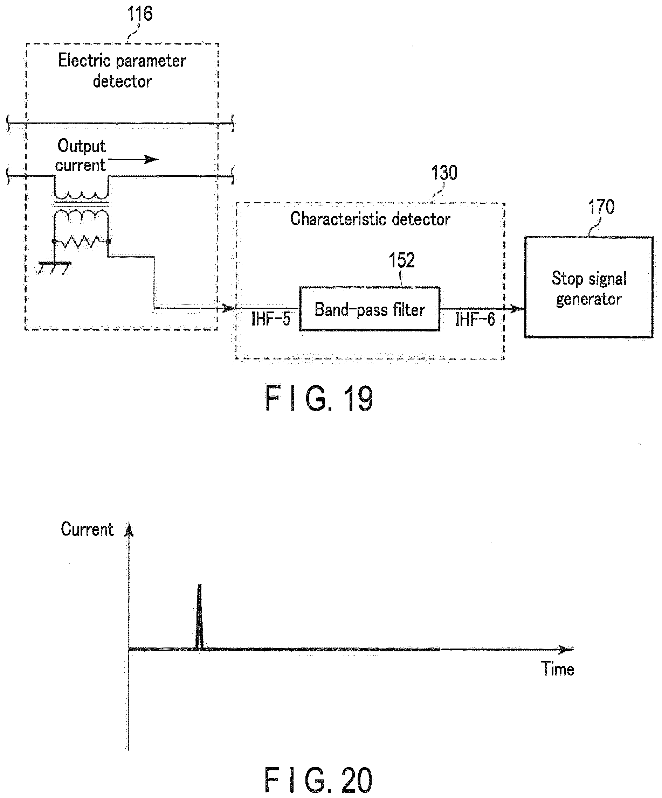

FIG. 19 illustrates the outline of a configuration example of the characteristic detector 130 according to the present embodiment. As shown in FIG. 19, the characteristic detector 130 comprises a band-pass filter 152. The band-pass filter 152 is a band-pass filter which passes signals of a frequency corresponding to a frequency of a characteristic current.

The fifth high-frequency current IHF-5, as a current used for detection retrieved at the electric parameter detector 116, is input to the characteristic detector 130. The fifth high-frequency current IHF-5 has a waveform like the one shown in FIG. 6 and FIG. 7, for example. Since the band-pass filter 152 passes signals of a frequency corresponding to a characteristic current, if the fifth high-frequency current IHF-5 as shown in FIG. 7, for example, is input to the band-pass filter 152, the sixth high-frequency current IHF-6 which passes the band-pass filter 152 will have a waveform like the one shown in FIG. 20.

The sixth high-frequency current IHF-6 which has passed the band-pass filter 152 is input to the stop signal generator 170. The stop signal generator 170 generates a stop signal when a signal including a peak as shown in FIG. 20 is found in the inputted sixth high-frequency current IHF-6, and outputs the stop signal. The configuration and operation of the power supply device 10 other than the above is the same as those of the first embodiment.

According to the present embodiment, similar to the first embodiment, the power supply device 10 detects a sign indicating a flow of an excessive current, and the output is stopped for a short period of time before an excessive current flows. As a result, a flow of an excessive current can be prevented. As a result, the power supply device 10 according to the present embodiment can control a current with high accuracy. According to the configuration of the present embodiment, fast and accurate characteristic current detection can be performed.

The present embodiment is of course applicable to the fourth to sixth modifications of the first embodiment.

### [Third Embodiment]

The third embodiment will be described. Herein, differences from the first embodiment will be described, and elements specified by the same reference numbers carry out the same operations, and a duplicate description of such elements will be omitted. In the first embodiment, the characteristic detector 310 monitors a current value to detect signs of applying of an excessive amount of power. On the other hand, the characteristic detector 130 according to the present embodiment detects signs of excessive power applying, using either a current value or a voltage value, whichever achieves a higher accuracy.

When the impedance of the circuit in which the high-frequency current that is output from the power supply device 10 flows is low, the output is a large current and a low voltage. In this case, the characteristic detector 130 monitors a current. On the other hand, when the impedance of the circuit in which the high-frequency current that is output from the power supply device 10 flows is high, the output is a small current and a high voltage. In this case, the characteristic detector 130 monitors a voltage.

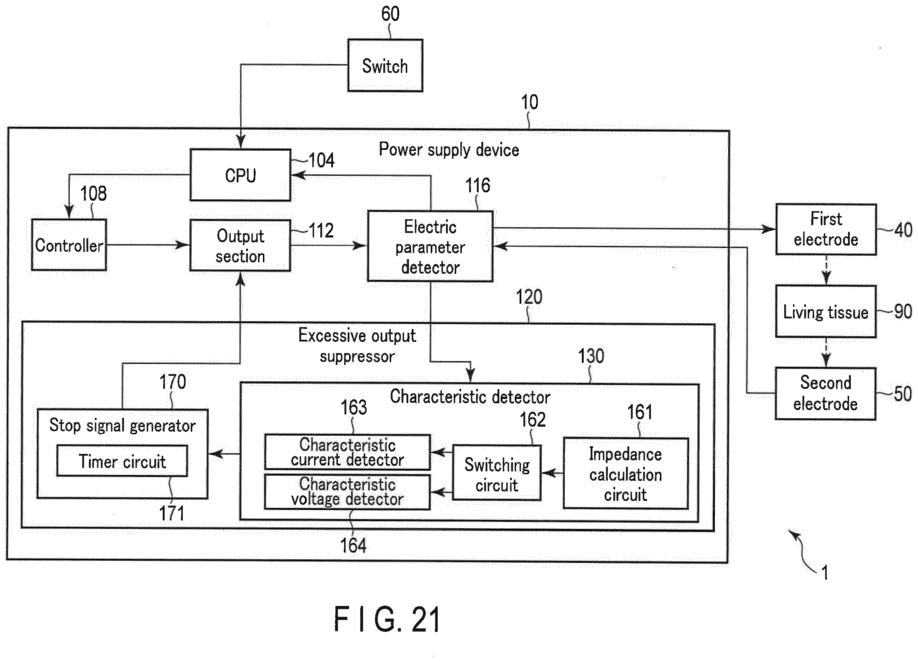

FIG. 21 illustrates the outline of the configuration example of the high-frequency treatment system 1 according to the present embodiment. As shown in FIG. 21, the high-frequency treatment system 1 of the present embodiment is different from the high-frequency treatment system 1 according to the first embodiment with respect to the configuration of the characteristic detector 130. The characteristic detector 130 according to the present embodiment comprises an impedance calculation circuit 161, a switching circuit 162, a characteristic current detector 163, and a characteristic voltage detector 164.

The impedance calculation circuit 161 calculates impedance of a circuit in which the high-frequency current that is output from the power supply device 10 flows. The impedance calculation circuit 161 outputs a value of the obtained impedance to the switching circuit 162. The switching circuit 162 determines whether monitoring of a current or monitoring of a voltage should be performed, in accordance with a value of the impedance calculated by the impedance calculation circuit 161. When monitoring of a current is performed, the switching circuit 162 has the characteristic current detector 163 detect a characteristic current. On the other hand, when monitoring of a voltage is performed, the switching circuit 162 has the characteristic voltage detector 164 detect a characteristic voltage.

The characteristic current detector 163 has a configuration same as the characteristic detector 130 according to the first embodiment or the modifications thereof, and detects a characteristic current. The characteristic current detector 163 outputs a signal indicating presence/absence of a characteristic current to the stop signal generator 170.

The characteristic voltage detector 164 performs monitoring of a voltage value. FIGS. 5 to 7 show a waveform of a current value obtained at the electric parameter detector 116; the waveform of a voltage value obtained at the electric parameter detector 116 also shows the same waveform as in FIGS. 5 to 7. The characteristic voltage detector 164 detects a characteristic voltage having a frequency higher than a fundamental frequency based on the waveform of the voltage value, similar to the characteristic current detector 163. The characteristic voltage detector 164 outputs a signal indicating presence/absence of a characteristic voltage to the stop signal generator 170. The configuration other than the above is the same as that of the first embodiment.

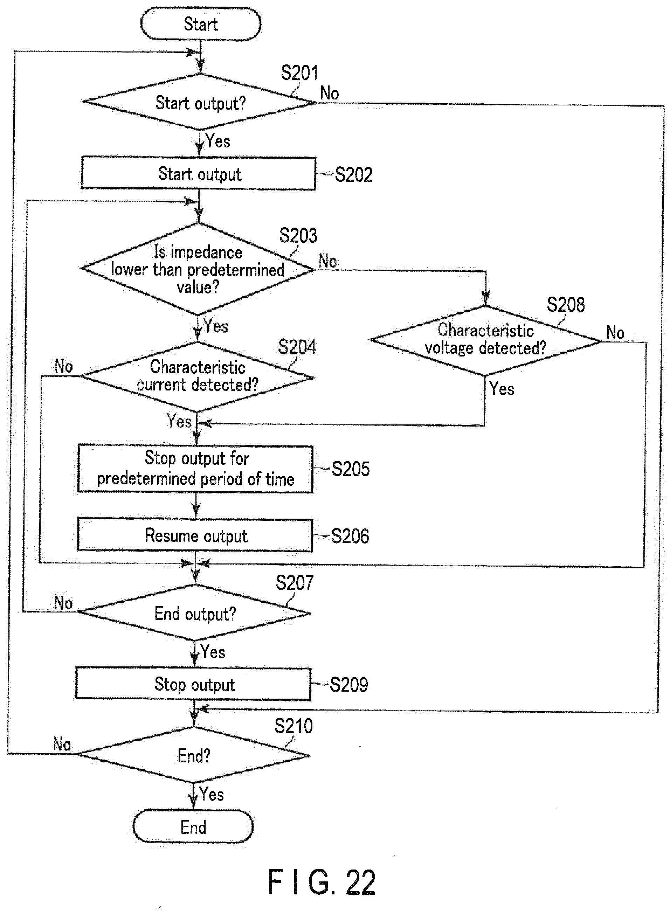

The operation of the power supply device 10 according to the present embodiment will be described with reference to the flowchart shown in FIG. 22. The process shown in FIG. 22 is started when the power supply device 10 is turned on, for example.

In step S201, the power supply device 10 determines whether the output should be started or not. In other words, the CPU 104, for example, determines whether the switch 60 is turned on or not. If the output is not started, the process proceeds to step S210, and the process in step S201 is repeated to wait, unless the power source is turned off as will be described later. When the output is started, the process proceeds to step S202.

In step S202, the power supply device 10 starts the output. In other words, the CPU 104 outputs information indicating that the output should be performed and information regarding an output current to the controller 108. The controller 108 controls the operation of the output section 112 based on the information obtained from the CPU 104. The output section 112 outputs a designated high-frequency current under the control of the controller 108. The outputted high-frequency power flows in the first electrode 40 and the second electrode 50 via the electric parameter detector 116.

In step S203, the power supply device 10 determines whether or not the impedance is lower than a predetermined threshold. In other words, the impedance calculation circuit 161 of the characteristic detector 130 calculates impedance of the circuit. The switching circuit 162 of the characteristic detector 130 determines whether or not the calculated impedance is lower than the predetermined threshold. The predetermined threshold is, for example, 1000 Ω or so. When the impedance is lower than the threshold, the process proceeds to step S204.

In step S204, the power supply device 10 determines whether or not a characteristic current is detected. In other words, the current used for detection flows in the characteristic current detector 163, and a signal processed by the characteristic current detector 163 is input to the stop signal generator 170. The stop signal generator 170 determines whether or not a characteristic current is detected based on the inputted signal. If no characteristic current is detected, the process proceeds to step S207. On the other hand, if a characteristic current is detected, the process proceeds to step S205.

In step S205, the power supply device 10 stops the output for a predetermined period of time. In other words, the stop signal generator 170 generates a stop signal, and outputs the stop signal to the output section 112. The period during which the stop signal is output is a predetermined period of time counted by the timer circuit 171. The output section 112 which has received the stop signal stops the output only for the period during which the stop signal is received.

In step S206, the power supply device 10 resumes the output. In other words, the stop signal generator 170 stops outputting the stop signal after outputting the stop signal for the above-mentioned predetermined period. As a result, the output section 112 resumes the output.

In step S207, the power supply device 10 determines whether or not to end the output. In other words, the CPU 104 determines whether the switch 60 is turned off or not. If the power supply device 10 determines to not end the output, the process returns to step S203. In other words, the power supply device 10 repeats the above-described process from step S203 to step S207. When, on the other hand, the power supply device 10 determines to end the output, the process proceeds to step S209.

If it is determined in step S203 that the impedance is not lower than the predetermined threshold, the process proceeds to step S208. In step S208, the power supply device 10 determines whether or not a characteristic voltage is detected. In other words, the voltage signal used for detection is input to the characteristic voltage detector 164, and a signal processed by the characteristic voltage detector 164 is input to the stop signal generator 170. The stop signal generator 170 determines whether or not a characteristic voltage is detected based on the inputted signal. If no characteristic voltage is detected, the process proceeds to step S207. On the other hand, if a characteristic voltage is detected, the process proceeds to step S205, and the output is stopped for a predetermined period of time.

In step S209, the power supply device 10 stops the output. In step S210, the power supply device 10 determines whether or not to end the process at the power supply device 10. If the power is turned off, for example, the process at the power supply device 10 is ended. If the process is not ended, the process returns to step S201. In other words, the power supply device 10 repeats the above-described process from step S201 to step S210. When the process is ended, on the other hand, the repeated process is ended.

According to the present embodiment, similar to the first embodiment, the power supply device 10 detects a sign indicating a flow of an excessive current, and the output is stopped for a short period of time before an excessive current flows. As a result, a flow of an excessive current can be prevented. When detecting a sign indicating a flow of the excessive current, switching between monitoring a current value or a monitoring a voltage value is performed based on impedance of the circuit. With the switching, the accuracy of detecting such a sign is improved in comparison to the case of only monitoring a current value as in the first embodiment. The present embodiment achieves the same advantageous effect as the first embodiment, besides the above.

The present embodiment is of course applicable to the modifications of the first embodiment and to the second embodiment.

Thus, when an excessive current is output from the power supply device 10, an excessive power having a second frequency higher than a fundamental frequency (a first frequency) of the outputted power is generated. The characteristic power may be detected as a characteristic signal based on a signal value of any of a current value, a voltage value, and a power value. In other words, any of a current value, a voltage value, or a power value may be monitored for detecting a characteristic power even in the first and second embodiments. As for the output from the power supply device 10, any procedures may be adopted from the control based on a current value, the control based on a voltage value, and the control based on a power value.

## Claims

1. A power supply device (10) for a high-frequency treatment instrument (41, 48) which performs high-frequency treatment on living tissue (90), the device (10) comprising:
an output section (112) which outputs power having a first frequency supplied to the high-frequency treatment instrument (41, 48),
wherein the device further comprises:
a characteristic detector (130) which detects a characteristic signal generated before the power outputted from the output section (112) becomes excessive, the characteristic signal having a second frequency higher than the first frequency and being included in a signal related to the power; and
a stop signal generator (170) which has a timer (171) for measuring a predetermined period of time of 50 milliseconds to 100 milliseconds and which outputs, for the predetermined period of time, a stop signal to stop or reduce an output of the power from the output section (112) when the characteristic signal is detected, wherein
the stop signal generator (170) stops outputting the stop signal after outputting the stop signal for the predetermined period of time so that the output section (112) resumes an output,
the output section (112) gradually increases the output after stopping the output for the predetermined period of time and resuming the output, so that the output section (112) reverts to an output level before stopping the output, and
the characteristic detector (130) compares a first signal which is the signal related to the power and a second signal which is a signal related to the power that has passed a low-pass filter that passes a signal having a frequency lower than the second frequency and, when a signal value of the first signal becomes greater than a signal value of the second signal, the characteristic detector (130) determines that the characteristic signal is detected.

2. The power supply device (10) according to claim 1, wherein the characteristic detector (130) compares the first signal and a third signal which is the second signal to which a positive bias is applied, and when the signal value of the first signal becomes greater than a signal value of the third signal, the characteristic detector (130) determines that the characteristic signal is detected.

3. The power supply device (10) according to claim 1, wherein the characteristic detector (130) compares the first signal and a fourth signal which is the second signal to which a negative bias is applied, and when the signal value of the first signal becomes lower than a signal value of the fourth signal, the characteristic detector (130) determines that the characteristic signal is detected.

4. The power supply device (10) according to claim 1, wherein the characteristic detector (130) compares the second signal and a fifth signal which is the first signal to which a negative bias is applied, and when a signal value of the fifth signal becomes greater than the signal value of the second signal, the characteristic detector (130) determines that the characteristic signal is detected.

5. The power supply device (10) according to claim 1, wherein the characteristic detector (130) compares the second signal and a sixth signal which is the first signal to which a positive bias is applied, and when a signal value of the sixth signal becomes lower than the signal value of the second signal, the characteristic detector (130) determines that the characteristic signal is detected.

6. The power supply device (10) according to claim 1, wherein the characteristic detector (130) comprises:
a current detector (163) which detects the characteristic signal based on a current value;
a voltage detector (164) which detects the characteristic signal based on a voltage value;
an impedance calculation circuit (161) which calculates impedance of a circuit based on the power; and
a switching circuit (162) which has the current detector (163) detecting the characteristic signal when the impedance is low, and which has the voltage detector (164) detecting the characteristic signal when the impedance is high.

7. A high-frequency treatment system (1) comprising:
the power supply device (10) according to claim 1; and
the high-frequency treatment instrument (41, 48) connected to the power supply device.

## Patentansprüche

1. Stromversorgungsvorrichtung (10) für ein Hochfrequenz-Behandlungsinstrument (41, 48), das Hochfrequenz-Behandlung von lebendem Gewebe (90) durchführt, wobei die Vorrichtung (10) umfasst:
einen Ausgabeabschnitt (112), der Leistung mit einer ersten Frequenz ausgibt, die dem Hochfrequenz-Behandlungsinstrument (41, 48) zugeführt wird,
wobei die Vorrichtung ferner umfasst:
einen Charakteristik-Detektor (130), der ein charakteristisches Signal erfasst, das erzeugt wird, bevor die von dem Ausgabeabschnitt (112) ausgegebene Leistung übermäßig wird, wobei das charakteristische Signal eine zweite Frequenz hat, die höher ist als die erste Frequenz, und in einem Signal enthalten ist, das sich auf die Leistung bezieht; und
einen Stoppsignalgenerator (170), der einen Zeitgeber (171) zum Messen einer vorbestimmten Zeitspanne von 50 Millisekunden bis 100 Millisekunden aufweist und der für die vorbestimmte Zeitspanne ein Stoppsignal ausgibt, um eine Ausgabe der Leistung von dem Ausgabeabschnitt (112) zu stoppen oder zu reduzieren, wenn das charakteristische Signal erfasst wird, wobei
der Stoppsignalgenerator (170) die Ausgabe des Stoppsignals nach Ausgabe des Stoppsignals für die vorbestimmte Zeitspanne stoppt, so dass der Ausgabeabschnitt (112) eine Ausgabe wieder aufnimmt,
der Ausgabeabschnitt (112) die Ausgabe allmählich erhöht, nachdem er die Ausgabe für die vorbestimmte Zeitspanne angehalten und die Ausgabe wieder aufgenommen hat, so dass der Ausgabeabschnitt (112) zu einem Ausgabepegel vor dem Anhalten der Ausgabe zurückkehrt, und
der Charakteristik-Detektor (130) vergleicht ein erstes Signal, das das auf die Leistung bezogene Signal ist, und ein zweites Signal, das ein auf die Leistung bezogenes Signal ist, das einen Tiefpassfilter passiert hat, der ein Signal mit einer Frequenz durchlässt, die niedriger als die zweite Frequenz ist, und wenn ein Signalwert des ersten Signals größer als ein Signalwert des zweiten Signals wird, bestimmt der Charakteristik-Detektor (130), dass das charakteristische Signal erfasst wird.

2. Stromversorgungsvorrichtung (10) nach Anspruch 1, wobei der Charakteristik-Detektor (130) das erste Signal und ein drittes Signal vergleicht, das das zweite Signal ist, an das eine positive Vorspannung angelegt wird, und wenn der Signalwert des ersten Signals größer wird als ein Signalwert des dritten Signals, bestimmt der Charakteristik-Detektor (130), dass das charakteristische Signal erfasst wird.

3. Stromversorgungsvorrichtung (10) nach Anspruch 1, wobei der Charakteristik-Detektor (130) das erste Signal und ein viertes Signal vergleicht, das das zweite Signal ist, an das eine negative Vorspannung angelegt wird, und wenn der Signalwert des ersten Signals niedriger als ein Signalwert des vierten Signals wird, bestimmt der Charakteristik-Detektor (130), dass das charakteristische Signal erfasst wird.

4. Stromversorgungsvorrichtung (10) nach Anspruch 1, wobei der Charakteristik-Detektor (130) das zweite Signal und ein fünftes Signal vergleicht, das das erste Signal ist, an das eine negative Vorspannung angelegt wird, und wenn ein Signalwert des fünften Signals größer als der Signalwert des zweiten Signals wird, bestimmt der Charakteristik-Detektor (130), dass das charakteristische Signal erfasst wird.

5. Stromversorgungsvorrichtung (10) nach Anspruch 1, wobei der Charakteristik-Detektor (130) das zweite Signal und ein sechstes Signal vergleicht, das das erste Signal ist, an das eine positive Vorspannung angelegt wird, und wenn ein Signalwert des sechsten Signals niedriger als der Signalwert des zweiten Signals wird, bestimmt der Charakteristik-Detektor (130), dass das charakteristische Signal erfasst wird.

6. Stromversorgungsvorrichtung (10) nach Anspruch 1, wobei der Charakteristik-Detektor (130) umfasst:
einen Stromdetektor (163), der das charakteristische Signal auf der Grundlage eines Stromwertes erfasst;
einen Spannungsdetektor (164), der das charakteristische Signal auf der Grundlage eines Spannungswertes erfasst;
eine Impedanzberechnungsschaltung (161), die die Impedanz einer Schaltung auf der Grundlage der Leistung berechnet; und
einen Schaltkreis (162), der den Stromdetektor (163) aufweist, der das charakteristische Signal erfasst, wenn die Impedanz niedrig ist, und der den Spannungsdetektor (164) aufweist, der das charakteristische Signal erfasst, wenn die Impedanz hoch ist.

7. Hochfrequenz-Behandlungssystem (1), das umfasst:
die Stromversorgungseinrichtung (10) nach Anspruch 1; und
das an die Stromversorgungseinrichtung angeschlossene Hochfrequenz-Behandlungsinstrument (41, 48).

## Revendications

1. Dispositif d'alimentation en électricité (10) pour un instrument de traitement à haute fréquence (41, 48) qui effectue un traitement à haute fréquence sur des tissus vivants (90), le dispositif (10) comprenant:
une section de sortie (112) qui délivre l'électricité ayant une première fréquence fournie à l'instrument de traitement à haute fréquence (41, 48),
dans lequel le dispositif comprend en outre :
un détecteur de caractéristique (130) qui détecte un signal caractéristique généré avant que l'électricité délivrée par la section de sortie (112) ne devienne excessive, le signal caractéristique ayant une seconde fréquence supérieure à la première fréquence et étant inclus dans un signal associé à l'électricité; et
un générateur de signal d'arrêt (170) qui a un temporisateur (171) destiné à mesurer une période de temps prédéterminée de 50 millisecondes à 100 millisecondes et qui délivre, pour la période de temps prédéterminée, un signal d'arrêt pour arrêter ou pour réduire une sortie de l'électricité provenant de la section de sortie (112) lorsque le signal caractéristique est détecté, dans lequel
le générateur de signal d'arrêt (170) arrête la sortie du signal d'arrêt après la sortie du signal d'arrêt pour la période de temps prédéterminée de sorte que la section de sortie (112) reprenne une sortie,
la section de sortie (112) augmente graduellement la sortie après l'arrêt de la sortie pour la période de temps prédéterminée et la reprise de la sortie, de sorte que la section de sortie (112) revienne à un niveau de sortie avant l'arrêt de la sortie, et
le détecteur de caractéristique (130) compare un premier signal qui est le signal associé à l'électricité et un deuxième signal qui est associé à l'électricité qui est passée dans un filtre passe-bas qui fait passer un signal ayant une fréquence inférieure à la seconde fréquence et, lorsqu'une valeur de signal du premier signal devient supérieure à une valeur de signal du deuxième signal, le détecteur de caractéristique (130) détermine que le signal caractéristique est détecté.

2. Dispositif d'alimentation électrique (10) selon la revendication 1, dans lequel le détecteur de caractéristique (130) compare le premier signal et un troisième signal qui est le deuxième signal auquel est appliquée une polarisation positive, et lorsque la valeur de signal du premier signal devient supérieure à une valeur de signal du troisième signal, le détecteur de caractéristique (130) détermine que le signal caractéristique est détecté.

3. Dispositif d'alimentation en électricité (10) selon la revendication 1, dans lequel le détecteur de caractéristique (130) compare le premier signal et un quatrième signal qui est le deuxième signal auquel est appliquée une polarisation négative, et lorsque la valeur de signal du premier signal devient inférieure à une valeur de signal du quatrième signal, le détecteur de caractéristique (130) détermine que le signal caractéristique est détecté.

4. Dispositif d'alimentation en électricité (10) selon la revendication 1, dans lequel le détecteur de caractéristique (130) compare le deuxième signal et un cinquième signal est le premier signal auquel est appliquée une polarisation négative, et lorsqu'une valeur de signal du cinquième signal devient supérieure à la valeur de signal du deuxième signal, le détecteur de caractéristique (130) détermine que le signal caractéristique est détecté.

5. Dispositif d'alimentation en électricité (10) selon la revendication 1, dans lequel le détecteur de caractéristique (130) compare le deuxième signal et un sixième signal qui est le premier signal auquel est appliquée une polarisation positive, et lorsqu'une valeur de signal du sixième signal devient inférieure à la valeur de signal du deuxième signal, le détecteur de caractéristique (130) détermine que le signal caractéristique est détecté.

6. Dispositif d'alimentation en électricité (10) selon la revendication 1, dans lequel le détecteur de caractéristique (130) comprend:
un détecteur de courant (163) qui détecte le signal de caractéristique sur la base d'une valeur de courant;
un détecteur de tension (164) qui détecte le signal de caractéristique sur la base d'une valeur de tension;
une unité de calcul d'impédance (161) qui calcule l'impédance d'un circuit sur la base de l'électricité; et
une circuiterie de commutation (162) qui amène le détecteur de courant (163) à détecter le signal caractéristique lorsque l'impédance est faible, et qui amène le détecteur de tension (164) à détecter le signal caractéristique lorsque l'impédance est élevée.

7. Système de traitement à haute fréquence (1) comprenant:
le dispositif d'alimentation en électricité (10) selon la revendication 1; et
l'instrument de traitement à haute fréquence (41, 48) connecté au dispositif d'alimentation électrique.
